# EUROPEAN PATENT APPLICATION

(11) **EP 2 567 710 A1**
(43) Date of publication of application: **13.03.2013**
(21) Application number: 11777307.7
(22) Date of filing: 03.05.2011
(51) Int. Cl.: A61K 47/08, A61K 47/30, A61K 9/10, A61P 31/10

(54) **AQUEOUS PHARMACEUTICAL SYSTEM FOR THE ADMINISTRATION OF DRUGS TO THE NAILS**

(30) Priority: 07.05.2010 ES 201000614
(71) Applicant: Universidade De Santiago De Compostela, 15782 Santiago de Compostela (ES)
(72) Inventor: OTERO ESPINAR, Francisco Javier, E-15782 Santiago de Compostela (ES); NOGUEIRAS NIETO, Luis, E-15782 Santiago de Compostela (ES); ANGUIANO IGEA, Felisa, E-15782 Santiago de Compostela (ES)
(74) Representative: ABG Patentes, S.L.
(86) International application number: PCT/ES2011/070312
(87) International publication number: WO 2011/138484

(57) **Abstract**

Aqueous pharmaceutical system for the administration of drugs to the nails. The invention relates to a topical preparation for treating diseases of the toenails and / or hands nails, based on a thermosensitive aqueous gel that at room temperature behaves as a dissolution and after application of nails, forming a hydrated layer, film or hydrogel from which release the active ingredients. The composition of the gel contains mostly water, a gel-forming polymer sensitive to changes in temperature, solubilizing agents and enhancers of absorption and / or penetration of the drug in nails.

## Description

### Field of the invention

The present invention relates to drug delivery systems with antifungal and/or antipsoriatic activity for the treatment of nail diseases. In particular, the invention relates to the preparation of the composition and its use in the manufacture of medicaments.

### Background of the invention

Pathological nails disorders may include relatively harmless conditions such as pigmentation changes, which commonly occur in smokers, discoloration associated with the use of certain systemically administered drugs, or increased in fragility (ie. by the continued use of detergents). Nevertheless, nails disorders may be more serious accompanied by painful, debilitating process, dystrophy, hypertrophy and inflammatory or infectious processes. These conditions can affect patients negatively from a physical standpoint and are accompanied by an important social and psychological component that can degrade the quality of life.

Onychomycosis (fungal infection that affects 3-10% of the population in Europe) and psoriasis (autoimmune illness, which is suffered by the 1-3 % of population) are the main diseases that alter nail plate. In psoriasis as onychomycosis, the promotion of the drug penetration (steroids and antifungal agents) by using appropriate formulations may improve drug efficacy with a consistent reduction of side effects. In this sense, the main strategies currently used to improve the effectiveness of local treatment are (Baran R, Tosti A. Topical treatment of nail psoriasis with a new corticoid-containing nail lacquer formation. Journal of Dermatological Treatment. 10, 201-204, 1999; Monti D, Saccomani L, Chetoni P, Burgalassi S, Saettone MF, Mailland F. In vitro transungual permeation of ciclopirox from a hydroxypropyl chitosan-based, water-soluble nail lacquer. Drug Development and Industrial Pharmacy. 1:11-17, 2005; Mohor 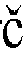 i 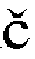 M, Torkar A, Friedrich J, Kristl J, Murdan S. A investigation into keratinolytic enzymes to enhance ungual drug delivery. International Journal of Pharmaceutics. 332, 196-201, 2007; Hui X, Shainhouse Z, Tanojo H, Anigbogu A, Markus GE, Maibach HI, Wester RC. Enhanced human nail drug delivery: nail inner drug content assayed by new unique method. Journal of Pharmaceutical Sciences. 91, 189-195, 2002; Hui X, Wester R.C, Barbadillo S, Lee C, Patel B, Wortzmman M, Gans EH, Maibach HI. Ciclopirox delivery into the human nail plate. Journal Of Pharmaceutical Sciences. 93, 2545-2548, 2004):
■ Use of more potent drugs.
■ Selection of drugs with suitable physicochemical properties to facilitate their penetration and diffusion into the nail and nail matrix;
■ Use of penetration and diffusion enhancers. (Eg, U.S. Patent No. 6,042,845, 6,159,977, 6,224,887, 6,391,879)
■ Design of formulations that bear high drug concentration and with prolonged residence time on the nail plate to further a controlled release of the drug. Sun et al. concluded the main problem of topical treatment is related to unsuitable formulation and poor drug release (Sun Y, Liu JC, Wang JC T, De Doncker P. Nail penetration. Ontopical Focus delivery of drugs for onychomycosis fungal Treatment. In: Bronaugh, RL, Maibach, HI (Eds), Percutaneus absorption. Drugs-Cosmetics-Mechanims-Methodology, 3rd Ed Marcel Dekker Inc, New York, pp. 759-787, 1999).

In this way some topical antifungal nail lacquers, which increase both the residence time of the vehicle and drug penetration, were marketed in recent years (i.e in Spain : flagstones 5% nail solution, Laboratories Galderma SA; Odenil Solution for nails, ISDIN, SA; CICLOCHEM Nail Novag Laboratories. In other countries: Penlac Nail Lacquer, Sanofi Aventis, Loceryl, Galderma Laboratories). Examples of new ungual drug delivery systems described in the patent literature are the following patent documents: US4.957.730 (describes a solution of 1-hydroxy-2-pyridone that generates a film water resistant ); US5.120.530 (amorolfine in a quaternary ammonium acrylic copolymer); US5.264.206 (tioconazole, econazole, oxiconazole, miconazole, tolnaftate, naftilina hydrochloride, included in a film insoluble in water); US5.346.692 (with urea and dibutyl phthalate as plasticizer) ; US5.487.776 (griseofulvin as colloidal suspension), US7.033.578 (nail varnish made from chitosan derivatives in volatile solvents) US5.464.610 (plaster of salicylic acid); WO 1999/39680 (nail lacquer with dioxanes, dioxolanes y acetals as penetration enhancers), US6.495.124 (antifungal nail lacquer elaborated with filmogenic polymers dispersed in organic solvents including cyclic lactones as plasticizer and penetration enhancers).

The effectiveness of nail lacquer as vehicles for topical administration of an antifungal agent, amorolfine, has already been described by Jean-Paul L. Marty, J. European Academy of Dermatology and Venereology, 4 (Suppl. 1), pp. S17-S21 (1995). However, nails lacquers are usually composed of polymers dispersed or dissolved in volatile organic solvents leading to the formation of high viscous, water-impermeable films on the nail plate surface as solvent evaporates. Nevertheless, the use of organic solvents exhibits important drawbacks such as their toxicity, irritation, low diffusion of drugs and enhancers and occlusive, harmful effects in fungal infections. With the aim of minimizing these effects, it has been proposed to replace organic nail lacquers with aqueous ones. In these systems water solutions or mixtures of water with cosolvents are used as drug vehicle in the preparation of nail lacquers (US2009/0175945, antipsoriatic nail lacquer containing mixtures of water and alcohol, nail polish EP039132 containing acrylic polymer dissolved in water; EP0627212 aqueous coating containing a polyurethane polymer film forming and an organic compound soluble perfluoroalkyl type; EP0648485 lacquer containing aqueous anionic polyester-polyurethane polymer in dispersed state, EP0943310 or US6238679 film-forming composition comprising an aqueous polyurethane dispersion and an agent plasticizer).

It is known that the efficacy of bioadhesive systems increase drug residence time on the skin and mucous membranes. Bioadhesive systems show also good biocompatibility and low toxicity so their use in nail delivery may also bring obvious advantages (Myoung Y, Choi HK. Permeation of ciclopirox across porcine hoof membrane: effect of pressure sensitive adhesives and vehicles. European Journal of Pharmaceutical Sciences. 20, 319-325, 2003). Most common nail penetration enhancers, such as urea or acetylcysteine, are molecules soluble in water, so it can be easily incorporated into the bioadhesive aqueous systems in large quantities. On the other hand, it has been shown that water hydrates and swells nail structures promoting the penetration and diffusion of drugs (Gunt HB, Kasting GB. Effect of hydration on the Permeation of ketoconazole through in vitro human nail plate. 32, 254-260, 2007; Gunt HB, Miller MA, Kasting GB. Water diffusivity in human nail plate. Journal of Pharmaceutical Sciences. Vol. 96, No. 12, 3352-3362, 2007). For these reasons different aqueous systems have been proposed as ungual delivery systems: gels (semisolid), hydrogels, creams or aqueous lacquers. Examples of patents are WO2009089361 (hydrogels containing several layers for controlling the release), US2009/0202602 (patches made from crosslinked hydrogels of alkyl-pyrrolidone) US5.391.367 (aqueous alcoholic gel with ticonazole) US5.696.105 (mometasone furoate cream).

However, one of the main drawbacks of the preparation of aqueous formulations is the low aqueous solubility of the antifungals and steroids that hinders their incorporation at high concentration and increases water layer resistance on drug diffusion into the nail. It has been shown that the incorporation of free cyclodextrins or cyclodextrins:polymers complexes increases drug aqueous solubility in the aqueous systems, increasing the effective drug load that can be included in soluble form and significantly reducing the strength of the aqueous layer (Bibby DC, Davies NM, Tucker IG. Mechanisms by which cyclodextrins modify drug release from polymeric drug delivery systems. International Journal of Pharmaceutics. 197, 1-11, 2000; Brewster ME, Loftsson T. Cyclodextrins as pharmaceutical solubilizers. Advanced Drug Delivery Reviews 59, 645-666, 2007).

### Brief description of the invention

The present invention provides a novel aqueous pharmaceutical system for the administration of drugs in the nails, stable topical formulation, long-lasting and effective for the treatment or prevention of fungal infections, psoriasis and other diseases of the nail as atopic dermatitis or *lichen planus.*

The invention provides a thermosensitive pharmaceutical system, which has the advantage of being liquid at room temperature and gelling once it is administered on the nail, forming an adherent hydrogel film on the nail plate. The system is sensible at the temperature forming a film on the surface of the nail which delivers active biological substances.

An additional advantage of the system of the invention is its aqueous nature, as it forms a hydrogel, it allows to maintain hydrated the zone of application because it contains water.

An additional advantage is that the system does not include organic solvents in its preparation neither in its final formulation.

Therefore, in one embodiment the invention relates to an aqueous pharmaceutical system for drug administration in nails characterized for being a liquid at room temperature and able to form a solid hydrogel at the body temperature, which comprises Pluronic F127NF, water, a penetration enhancer and at least one biologically active substance.

In another embodiment, the invention relates to a method to prepare an aqueous pharmaceutical system, as defined above, which comprises dispersing or dissolving the Pluronic F127NF, a penetration enhancer and at least one biologically active substance in water.

In another embodiment, the invention relates to the use of an aqueous pharmaceutical system, as described above, for the preparation of a medicament for the treatment of fungal infections of the nails, nail psoriasis and other diseases of the nails as atopic dermatitis or *lichen planus.*

### Detailed description of the invention

The term "hydrogel" refers to a swollen three-dimensional macromolecular structure in an aqueous medium which is insoluble in these medium.

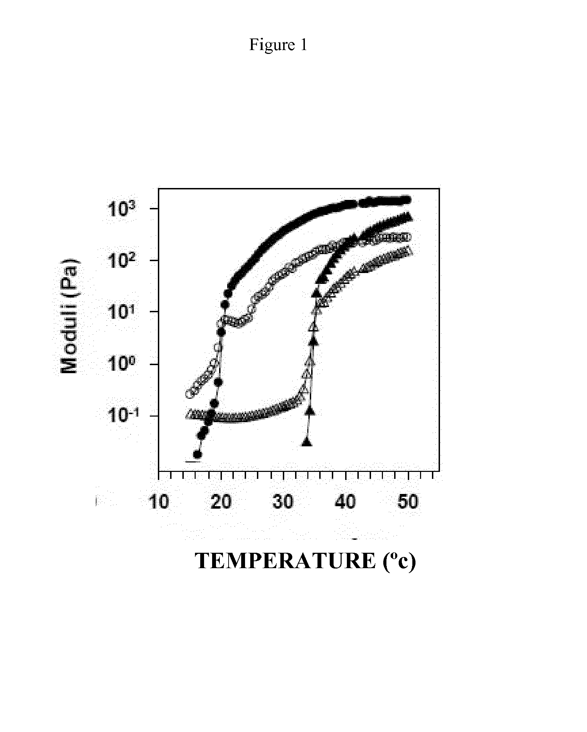

The term "thermosensitive polymer" refers to a polymer which is able to respond to a change in temperature and its response is a variation in its physical or physico-chemical properties. Hydrogels made from these thermosensitive materials exhibit a phase transition mediated by temperature that causes changes in their volume and rheological properties such as viscosity, consistency and viscoelasticity. Pluronic F127NF is a thermosensitive polymer because it undergoes a transition from solution to gel at temperatures close to the body temperature, forming structured and consistent hydrogels (Figure 1).

F127NF Pluronic, marketed by BASF, is a block copolymer of ethylene oxide and propylene oxide represented by the formula HO(C₂H₄O)ₐ(C₃H₆O)_{b}(C₂H₄O)ₐH where *a* is 101 and b is 56, characterized by a molecular weight of 12600, viscosity of 3100, melting point of 56 °C and solubility in water at 25 °C above 10%.

The term "room temperature" is the average temperature at which the being human feels comfortable, it can be a range between 18 and 27°C, more particularly between 20 and 25°C.

The term "body temperature" is the average temperature of the human body in healthy conditions, it comprises a range between 36 and 37,5°C.

In a particular embodiment of the invention the F127NF Pluronic polymer is incorporated in the aqueous pharmaceutical system in concentrations ranging between 10% and 40% by weight relative to the system; more particularly, between 15 and 20% by weight.

The term "penetration enhancer" refers to a substance used to enhance and facilitate the penetration and diffusion of biologically active substances through the nail matrix. Penetration enhancers are known compounds which purpose is to reduce sulfhydryl bridge bonds of keratin, such as cysteine, N-acetylcysteine, thioglycolic acid, sodium sulfite, keratinase, hydrogen peroxide, urea or mixtures thereof.

In a particular embodiment, N-acetylcysteine has been selected as penetration enhancer.

In another particular embodiment, the penetration enhancer is at a rate between 1% and 15% by weight.

In a particular embodiment, the aqueous pharmaceutical systems of the invention, as defined above, can additionally comprise a solubilizing agent.

The term "solubilizing agent" refers to substances that are added to the solution to increase the aqueous solubility of biologically active substances. The addition of solubilizing agents can increase the effective dose of biologically active substances in the system reducing the resistance of aqueous layer diffusion. For the invention it is particularly interesting the solubilizing agents selected from cyclodextrins and their derivatives, and hydrophilic polymers.

In a particular embodiment the cyclodextrins are selected from: α-, β-, and γ-cyclodextrin and their mixtures; α-, β-, and γ-alkyl-cyclodextrins and their mixtures; α-, β-, and γ-hydroxyalkyl-cyclodextrins as hydroxyethyl-β-cyclodextrin, hydroxypropyl-β-cyclodextrin, and hydroxypropyl-y-cyclodextrin, and their mixtures; α-, β-, and γ-sulfoalkyl-ether cyclodextrins as sulfobutylether-β-cyclodextrin and their mixtures; α-, β-, and γ- branched cyclodextrins with one or two glucosyl or maltosyl residues and their mixtures; and α-, β-, and γ-alkylcarboxyalkyl-cyclodextrins and their mixtures; or their mixtures in proportions from 0,1 % to 50%, over the total of the dissolution.

The term "alkyl" is referred to (C1-C6) lineal or branched alkyl groups.

In a more particular embodiment, the solubilizing agent is selected from hydroxypropyl-beta-cyclodextrin or beta-cyclodextrin or their derivatives partially methylated.

In a particular embodiment, hydrophilic polymers are selected from poloxamers, poloxamines, urea, polyethylene glycols, polyvinylpyrrolidone, polysorbates or polyvinyl alcohol.

In a particular embodiment, the solubilizing agents are in a ratio of 1% and 15% by weight.

Additionally the solution can also contain other physiologically acceptable additives such as acids, bases, or pH buffering systems.

In a particular embodiment, the invention refers to an aqueous pharmaceutical system, as defined above, comprising Pluronic F127NF, N-acetylcysteine, water and a biologically active substance. In a more particular embodiment, the biologically active substance is selected from triamcinolone and ciclopirox, and their salts. In another particular embodiment, additionally the system comprises hydroxypropyl-beta-cyclodextrin or beta-cyclodextrin, or their derivatives partially methylated.

In another particular embodiment, the invention refers to an aqueous pharmaceutical system, as defined above, comprising Pluronic F127NF between 10 and 40% by weight, an enhancer of penetration between 1 and 15% by weight, a substance biologically active between 0.01 and 100 mg / mL. In a more particular embodiment, additionally the system comprises a solubilizing agent between 1 and 15% by weight.

In another embodiment, the invention refers to a method for the preparation of the systems, as defined above, which comprises dispersing or dissolving Pluronic F127NF, a penetration enhancer and at least one biologically active substance in water.

This is a simple method, based on the dispersion and dissolution of the components in the aqueous medium. The method involves a single stage and does not have environmental or toxicological problems associated with the use of organic solvents neither the presence of residues of these solvents in the final product. The dispersion or dissolution of the components does not require any particular order.

In a particular embodiment, the system, as defined above, comprise additionally a solubilizing agent.

The term "biologically active substance" refers to any substance that is used to treat, cure or prevent fungal infections of the nails, nail psoriasis and other diseases of the nails as atopic dermatitis or *lichen planus.* When one or more biologically active substances are incorporated to the system of the invention, those are dispersed at the molecular level, particle level, forming complexes with components of the solution or included into systems to improve their solubility or control their release. The system of the invention is suitable for incorporating biologically active substances regardless their solubility properties. When the biologically active substance has low solubility in water, then the system must incorporate a solubilizing agent, as defined above, particularly when the active ingredient is a low water soluble steroidal anti-inflammatory.

In a particular embodiment, the biologically active substances are selected from steroidal anti-inflammatory and antifungal drugs.

In a particular embodiment, the antifungal drug is selected from the group consisting of polyenes, allylamines, imidazoles, triazoles such as econazole, ciclopirox, undecylenic acid and amorolfine, and their salts.

In another particular embodiment, the steroidal anti-inflammatory drug is selected from the group consisting of hydrocortisone, triamcinolone, betamethasone, clobestol, and their salts.

In a particular embodiment of the invention, the proportion of the biologically active substance is between 0.01 and 100 mg / mL.

In another embodiment, the invention refers to a pharmaceutical composition comprising the previously described system. The pharmaceutical composition can be applied to the nails by deposition, spraying, atomization, misting and / or immersion.

### Description of the figures

**Figure 1****.** Elastic modulus (closed symbols) and viscous modulus (open symbols) of 15% aqueous dispersions of Pluronic F127NF, without (circles) or with (triangles) 5% of partially methylated beta-cyclodextrin (MBCD), obtained using an AR-1000N Rheolyst rheometer (TA Instruments, Newcastle , UK), for different angular frequency at a temperature of 25 ° C (left graphs) or in function of the temperature for a frequency of 0.5 rad / s (right graphs). At low temperatures the system behaves like a liquid and increasing temperature the system becomes a structured hydrogel with high consistency. The sol-gel transition temperature increases by incorporating MBCD, (34-35 °C for F127NF Pluronic 15% and 5% of MBCD solutions).

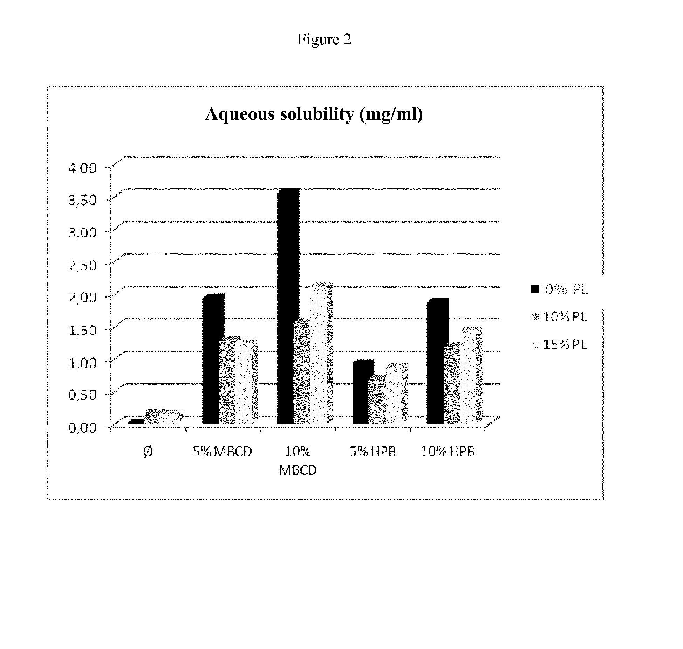

**Figure 2****.** Triamcinolone acetonide solubility values in systems with different proportions of Pluronic F127NF (PL), hydroxypropyl-β-cyclodextrin (HPB) or partially methylated β-cyclodextrin (MBCD). The N-acetylcysteine did not alter the solubility in these systems. Φ = no cyclodextrin

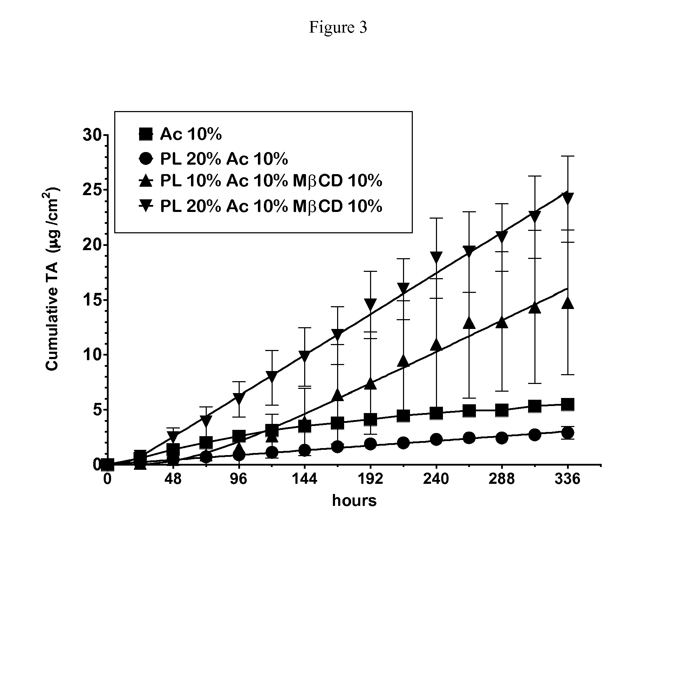

**Figure 3****.** Penetration profiles of triamcinolone acetonide through calf hoof from solutions of triamcinolone acetonide in the presence of 10% N-acetylcysteine (AC) and thermosensitive systems prepared with Pluronic F127NF (PL), with and without partially methylated beta-cyclodextrin (MBCD). We did not detect the penetration of triamcinolone acetonide from the solutions in the absence of N-acetylcysteine.

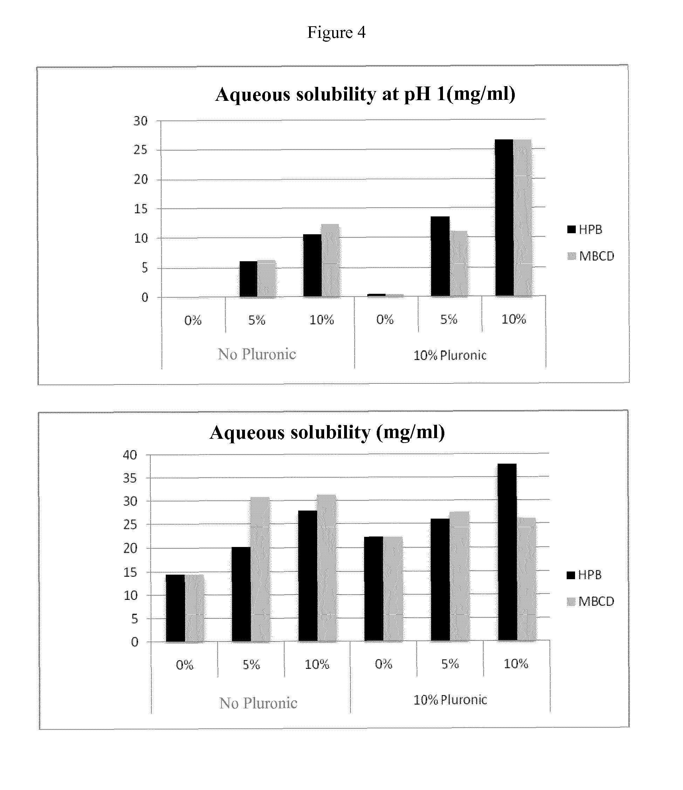

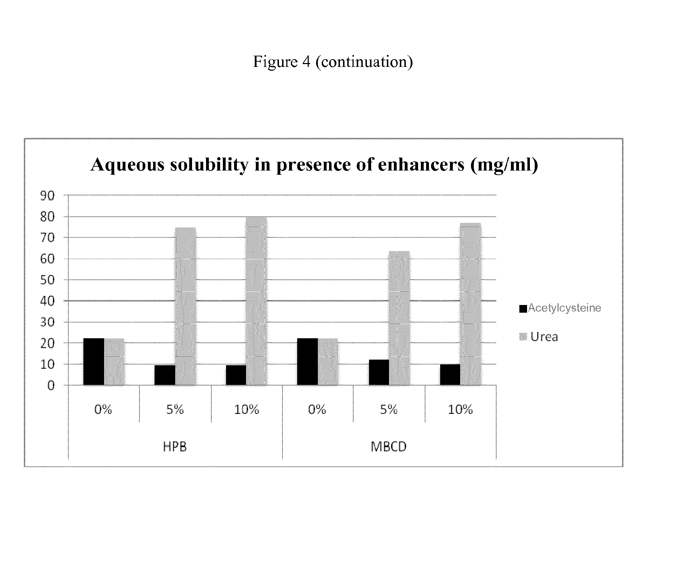

**Figure 4****.** Ciclopirox olamine solubility values in systems with different proportions of Pluronic F127NF (PL), hydroxypropyl-β-cyclodextrin (HPB) or partially methylated β-cyclodextrin (MBCD). The presence of N-acetylcysteine or urea significantly influences the solubility of this drug in these systems. Φ = no cyclodextrin.

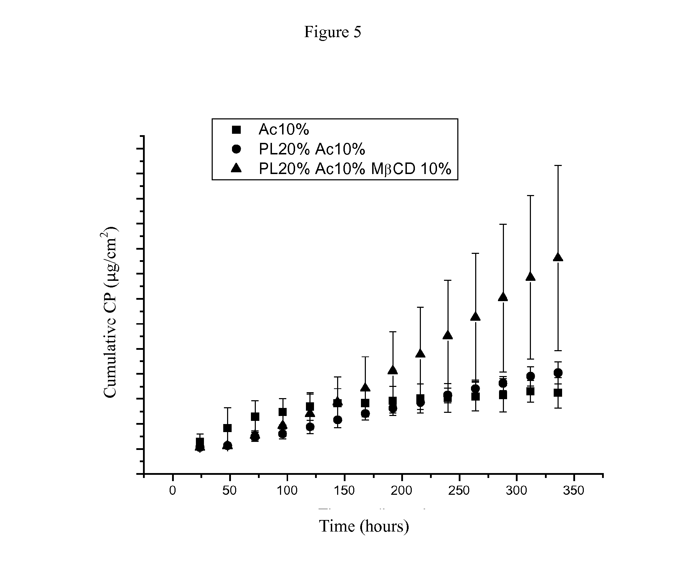

**Figure 5****.** Penetration profiles of ciclopirox olamine through calf hoof from ciclopirox olamine solutions in the presence of 10% N-acetylcysteine (AC) and thermosensitive systems prepared with Pluronic F127NF (PL), with and without partially methylated beta-cyclodextrin (MBCD). We did not detect drug diffusion and penetration from the solutions in the absence ofN acetylcysteine.

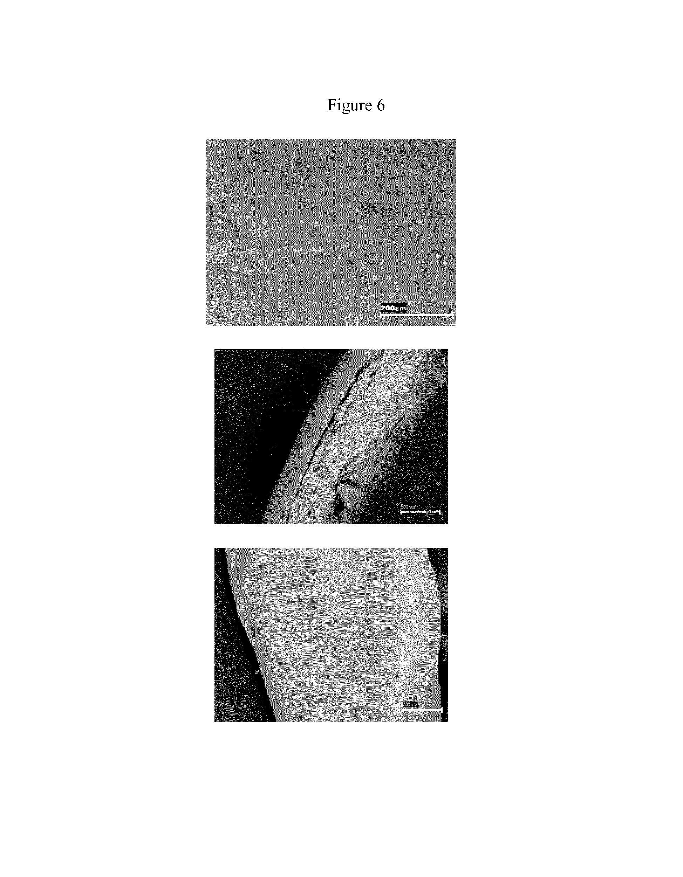

**Figure 6****.** SEM microphotographs of the film which is formed over the nail once the thermosensible system is applied. Above: view of the surface of the nail; Middle: view of the film formed over the surface of the nail; Below: cross-section which shows the film adherent to the surface of the nail.

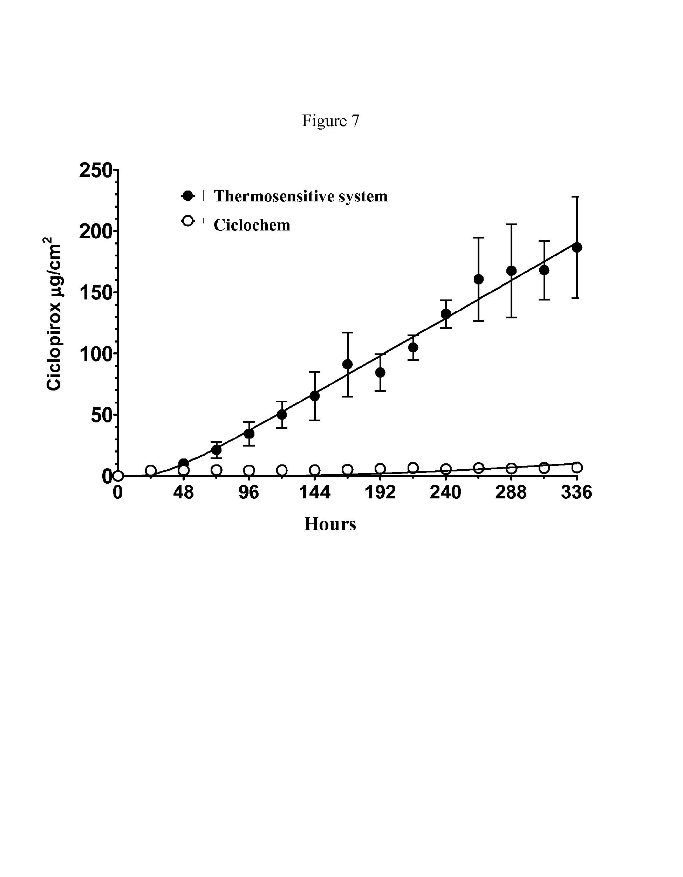

**Figure 7****.** Penetration of ciclopirox through human nail from the thermosensitive system comprising 0.37% of ciclopirox olamine and the formulation of reference with 8% of ciclopirox (Ciclochem^{®} nails solution).

For a better understanding of the invention, we provide the following examples, but they not limit the present invention.

### Example 1. Preparation of thermosensitive systems containing triamcinolone acetonide and Pluronic F127NF. Rheological study of the sol-gel transition of Pluronic F127NF dispersions with or without MBCD. Study of triamcinolone acetonide loading in the systems by using hydroxypropylated cyclodextrins (hydroxypropyl-β-cyclodextrin) or partially methylated beta-cyclodextrin. Penetration studies through the nail from the systems containing partially methylated cyclodextrin as a solubilizing agent.

The effect of temperature on the elastic modulus and viscosity of dispersions of Pluronic F127NF in the presence and absence of MBCD was assessed in triplicate in an AR-1000N rheometer Rheolyst Rheometer (TA Instruments, Newcastle, UK) with an analyzer AR2500 and a plate Peltier plate with geometry of 6 cm diameter and 2.1 degrees. The trial was conducted at 0.1 rad / s to 15 °C to 50 °C with a heating rate of 3 °C / min. Liquid paraffin was added around the cell sample to prevent evaporation of samples. Frequency experiments were performed at 25 ° C from 0.05 to 50 rad / s to 0.1 Pa.

Aqueous dispersions of Pluronic F127NF containing a 0.10 and 15%, were prepared by stirring the polymer in water at 4 °C to study the incorporation of the drug. Hydroxypropyl-β-cyclodextrin or partially methylated β-cyclodextrin at concentrations of 0, 0.5% and 10% were also incorporated. Triamcinolone acetonide was added in amounts that far exceeded its aqueous solubility and was stirred at 25 °C for a week. The amount of dissolved triamcinolone was determined spectrophotometrically after filtering the dispersions through a membrane filter of 0.45 micron pore size. The results are shown in Figure 1.

For the preparation of thermosensitive systems used in penetration studies through the nail we proceeded as it follows:
1.- Dispersion of the Pluronic F127NF at 10 or 20% (depending on the formulation) in cold water with magnetic stirring until complete dissolution.
2.- Incorporation of triamcinolone acetonide in quantities for concentrations close to saturation (see Figure 1).
3.- Addition ofN-acetylcysteine until 10%.

In the formulations containing partially methylated β-cyclodextrin, it was incorporated into the system at concentrations of 10% prior to the dissolution of triamcinolone acetonide in order to increase aqueous concentration of the drug (see Figure 1).

To perform the penetration studies, cylindrical thin slices of calf hoof membrane (0.8 - 1 mm) were used. The bovine hoof samples were placed using two Teflon adapters between the donor and acceptor compartment of vertical Franz-Chien penetration cells. In the receptor compartment, phosphate buffer (pH 7.4) was added and maintained at 37°C. 1g of the thermosensitive systems was placed into the acceptor compartment. Samples from receptor solution were collected at scheduled time, determining the concentration of triamcinolone acetonide by HPLC. The results were normalized by the diffusion surface and the thickness of the calf hoof slides used. The profiles obtained are shown in Figure 2.

### Example 2. Preparation of thermosensitive systems containing ciclopirox olamine and Pluronic F127NF. Study of ciclopirox olamine loading in the presence and absence of N-acetylcysteine or urea, employing hydroxypropyl-β-cyclodextrin or partially methylated β-cyclodextrin. Penetration studies on the nail from the systems containing partially methylated β-cyclodextrin as a solubilizing agent.

Dispersions of Pluronic F127NF (0, 10 and 15%) were prepared incorporating hydroxypropyl-β-cyclodextrin or partially methylated β-cyclodextrin at concentrations of 0, 0.5% and 10%. Ciclopirox olamine was added in amounts that far exceeded its aqueous solubility and was stirred at 25 °C for a week. The amount of dissolved ciclopirox was determined spectrophotometrically after filtering the dispersions. Pluronic F127NF dispersions containing 10% of hydroxypropyl-β-cyclodextrin or partially methylated β-cyclodextrin (5% or 10%) and N-acetylcysteine (5-10%) or urea (10-20%) has been studied. The results are shown in Figure 3.

Thermosensitive systems used in penetration studies were prepared as it follows:
1.- Dispersion of the Pluronic F127NF to 20% in cold water with magnetic stirring until complete dissolution.
2.- Ciclopirox olamine was incorporated in adequate quantities for concentrations close to saturation (see Figure 3).
3.- Dispersion of N-acetylcysteine at a concentration of 10%.

In the formulations prepared with partially methylated β-cyclodextrin, it was incorporated into the system at a concentration of 10% prior to the dissolution of triamcinolone acetonide, allowing soluble increasing doses of the drug (see Figure 3).

Penetration studies were performed with cylindrical thin slices of calf hoof membrane (0.8 - 1 mm). The bovine hoof samples were placed using two Teflon adapters between the donor and acceptor compartment of vertical Franz-Chien penetration cells. In the receptor compartment, phosphate buffer (pH 7.4) was added and maintained at 37°C. 1g of the thermosensitive systems was placed into the acceptor compartment. Samples of 400µL from receptor solution were collected at scheduled time, determining the concentration of triamcinolone acetonide spectrophotometrically. The medium extracted was replaced with the same amount of buffer. The results were normalized by the diffusion surface and the thickness of the calf hoof slides used. The profiles obtained are shown in Figure 4.

### Example 3. Preparation of thermosensitive systems containing 0.37% of ciclopirox olamine, 20% of Pluronic F127NF, 10% of partially methylated cyclodextrins and 10% of N-acetylcysteine. Study of the capacity of forming a film on the nail. Penetration studies on human nail and comparative with commercial formulation Ciclochem^{®}.

The polymer Pluronic F127NF to 20% was dissolved in cold water with magnetic stirring until complete dissolution. Then, partially methylated beta-cyclodextrin to 10%, ciclopirox olamine to 0.37 % (P/P) and N-acetylcysteine to 10% were added and finally the solution is filtered through a membrane filter of 0.45 micron pore size.

The study was performed with human nails from voluntaries between 20 and 30 years old. The nail samples were carefully cleaned, washed with water, dried at room temperature and stored at room temperature until they were used. The samples for penetration studies had a length of 8 mm. In the studies of the formation of the film, the samples had a length of 3 mm.

The formulation was placed on the top of the nail samples with a brush standing at room temperature, to perform the studies of the film formation. Figure 6 shows the scanning electron microscope microphotographs obtained with a Leo VP-435 SEM (Leo Electron Microscopy, UK). These microphotographs show that once the water is absorbed by the nail, a homogeneous polymeric film is formed on the surface of the nail, from which the drug is delivered.

To perform the penetration studies, nail samples were placed were placed using two Teflon adapters between the donor and acceptor compartment of vertical Franz-Chien penetration cells. In the receptor compartment, phosphate buffer (pH 7.4) was added and maintained at 37°C. 1g of the thermosensitive systems or the reference was placed into the acceptor compartment. Samples of 400µL from receptor solution were collected at scheduled time, determining the concentration of ciclopirox olamine spectrophotometrically. The medium extracted was replaced with the same amount of buffer. The results were normalized by the diffusion surface. The profiles obtained are shown in Figure 7.

The reference for the penetration studies was CICLOCHEM^{®} nails solution (Laboratorios Ferrer) which includes 80 mg of Ciclopirox by gram of solution and it is elaborated with the following excipients: methoxyethene, polymer with 2-butenoic acid, monobutyl ester, (Gantrez ES-435), ethyl acetate, 2-propanol.

Penetration studies show that the thermosensitive system provides a higher flux of ciclopirox through the nail than the reference, which indicates that the penetration of the drug is higher and faster when the thermosensitive system of the invention is used.

## Claims

1. Aqueous pharmaceutical system for drug administration in nails characterized for being a liquid at room temperature and able to form a solid hydrogel at the body temperature, which comprises Pluronic F127NF, water, a penetration enhancer and at least one biologically active substance.

2. Aqueous pharmaceutical system according to claim 1, wherein the Pluronic F127NF is in concentrations ranging between 10% and 40% by weight relative to the system.

3. Aqueous pharmaceutical system, according to previous claims, wherein the penetration enhancer is between 1% and 15% by weight.

4. Aqueous pharmaceutical system, according to previous claims, which further comprises a solubilizing agent.

5. Aqueous pharmaceutical system, according to previous claims wherein the biologically active substance is selected from steroidal anti-inflammatory and antifungal drugs.

6. Aqueous pharmaceutical system according to claim 1 and 5, wherein the biologically active substance is between 0.01 and 100 mg / mL.

7. Method to prepare an aqueous pharmaceutical system, as defined in claim 1, which comprises dispersing or dissolving the Pluronic F127NF, a penetration enhancer and at least one biologically active substance in water.

8. Method according to claim 7, which further comprises a solubilizing agent.

9. Use of an aqueous pharmaceutical system, as described in claim 1, for the preparation of a medicament for the treatment of fungal infections of the nails and nail diseases.

10. Use according to claim 9, wherein the diseases of the nails are selected from atopic dermatitis or *lichen planus.*
